# EUROPÄISCHE PATENTSCHRIFT

(11) **EP 1 611 083 B1**
(45) Veröffentlichungstag und Bekanntmachung des Hinweises auf die Patenterteilung: **03.10.2007**
(21) Anmeldenummer: 04719953.4
(22) Anmeldetag: 12.03.2004
(51) Int. Cl.: C07C 69/94, C07D 263/32

(54) **SYNTHESE VON 2-CHLORMETHYL-6-METHYLBENZOESÄUREESTERN**
SYNTHESIS OF 2-CHLOROMETHYL-6-METHYLBENZOIC ACID ESTERS
SYNTHESE D'ESTERS DE L'ACIDE 2-CHLOROMETHYL-6-METHYLBENZOIQUE

(30) Priorität: 25.03.2003 DE 10313228
(43) Veröffentlichungstag der Anmeldung: 04.01.2006
(73) Patentinhaber: Sanofi-Aventis Deutschland GmbH, 65929 Frankfurt am Main (DE)
(72) Erfinder: MAIER, Claus-Jürgen, 65926 Frankfurt am Main (DE); METZENTHIN, Tobias, 65719 Hofheim (DE); GRAESER, Joachim, 65451 Kelsterbach (DE); BICKER, Richard, 68358 Liederbach (DE); MANERO, Javier, 65832 Liederbach (DE)
(86) Internationale Anmeldenummer: PCT/EP2004/002579
(87) Internationale Veröffentlichungsnummer: WO 2004/085377

(56) Entgegenhaltungen:
- WO-A-00/64876
- WO-A-00/64888
- WO-A-03/020269

## Beschreibung

2-Halogenalkylbenzoesäurederivate werden als Bausteine für die Synthese von pharmazeutischen Wirkstoffen eingesetzt. Für den Einsatz ist es aus verschiedenen Gründen erstrebenswert, lagerstabile Verbindungen zu haben, die sich zudem einfach herstellen und reinigen lassen. Zu diesen Gründen zählen z.B. die Sicherstellung einer konstanten Qualität, die Vermeidung von häufigen Kontrollen der Materialien zur Protokollierung der Produktqualität, die Vermeidung der Notwendigkeit von Kühllagern und/oder Kühltransporten, leichtes Einfüllen in Produktionsanlagen sowie einfache Reinigung von benutzten Behältern.

2-Brommethyl-6-methylbenzoesäureester (A1 und A2) sind beispielsweise bekannt aus WO 00/64888 (R = iBu (A1)) und WO 00/64876 (R = Me (A2)). Diese Verbindungen sind bei Raumtemperatur nicht lagerstabil, da sie spontan zum Lacton (B) cyclisieren und dabei bekanntermaßen mutagene Alkylbromide als Nebenprodukt freisetzen.

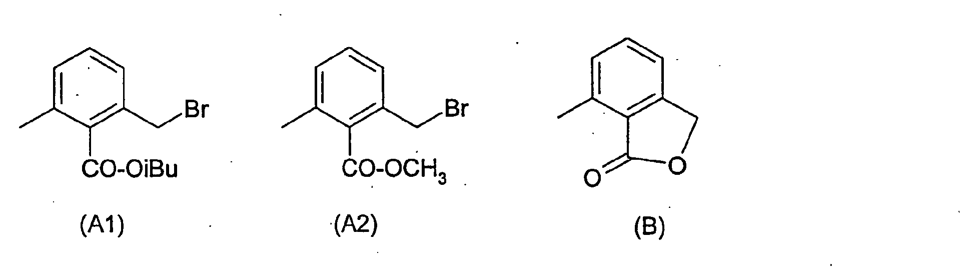

Ein Einsatz dieser thermolabilen Substanzen im technischen Maßstab ist daher mit arbeitshygienischen Risiken, Schwierigkeiten und zusätzlichen Kosten verbunden.

Die 2-Brommethyl-6-methylbenzoesäureester sind u. a. interessant als Ausgangsmaterialien für die Herstellung von PPAR Agonisten wie z.B. in WO 00/64888, WO 00/64876 und WO 03/020269 beschrieben. Insbesondere seien hier die Verbindungen der Formel (C) genannt: 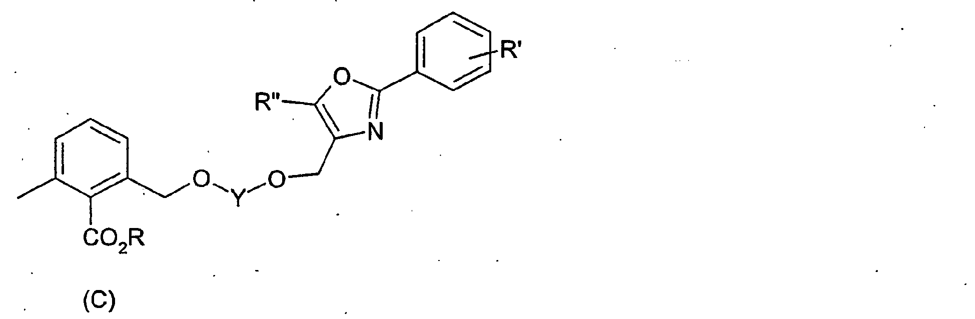 mit
- R: H, C₁-C₁₂-Alkyl, C₃-C₈-Cycloalkyl, C₆-C₁₂-Aryl, C₁-C₄-Alkyl-C₆-C₁₂-Aryl oder C₅-C₁₀-Heteroaryl, wobei in Alkyl und Cycloalkyl ein oder mehrere CH₂-Gruppen durch -O- ersetzt und Alkyl, Cycloalkyl und Aryl durch Halogen, substituiert sein können,
- Y: -(CH₂)₃-, 1,3-Phenylen, 1,3-Cyclohexandiyl;
- R': H, F, Br, CF₃, (C₁-C₆)-Alkyl, O-(C₁-C₆)-Alkyl, Phenyl;
- R": H, (C₁-C₆)-Alkyl, (C₁-C₃)-Alkyl-Phenyl, (C₅-C₆)-Cycloalkyl. Phenyl, CF₃.

Bevorzugt seien die Verbindungen der Formel (C) genannt, in denen der Phenylring durch R' in m- oder p-Stellung substituiert ist.

Es stellte sich nun die Aufgabe, stabilere Verbindungen als (A1) und (A2) zu finden, die die oben geschilderten Nachteile nicht aufweisen. Zusätzlich sollten die Verbindungen, die bei ihrer Herstellung auch aus einem Rohprodukt mit noch nicht ausreichender Reinheit anfallen können, im Gegensatz zu den Verbindungen der Formeln (A1) und (A2), aufreinigbar sein.

Dies gelang mit den nachstehend beschriebenen Verbindungen der Formel (I).

Gegenstand der Erfindung sind die Verbindungen der Formel (I) 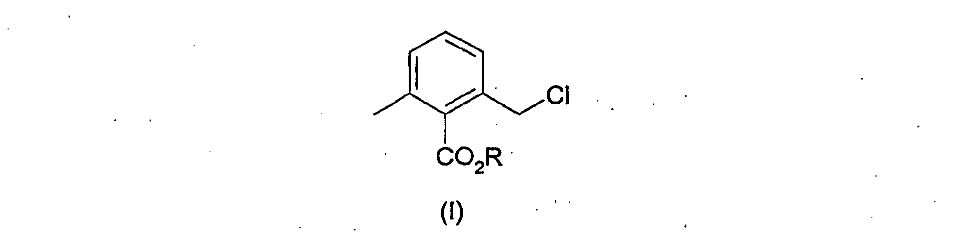 worin
- R: H, C₁-C₁₂-Alkyl, C₃-C₈-Cycloalkyl, C₆-C₁₂-Aryl, C₁-C₄-Alkyl-C₆-C₁₂-Aryl oder C₅-C₁₀-Heteroaryl bedeutet und wobei in Alkyl und Cycloalkyl ein oder mehrere CH₂-Gruppen durch -O- ersetzt und Alkyl, Cycloalkyl und Aryl durch Halogen, substituiert sein können.

Bevorzugt sind die Verbindungen der Formel (I), worin
- R: C₁-C₈ Alkyl, C₃-C₆-Cycloalkyl oder C₁-C₄-Alkyl-C₆-C₁₂-Aryl bedeutet, die gegebenenfalls durch Halogen substituiert sind und wobei ein oder zwei CH₂-Gruppen durch -O- ersetzt sein können.

Besonders bevorzugt sind die Verbindungen der Formel (1), worin
- R: C₁-C₆ Alkyl oder C₁-C₄-Alkyl-C₆-C₁₂-Aryl ist, die gegebenenfalls durch Halogen substituiert sind und wobei eine CH₂-Gruppe durch -O- ersetzt sein kann.

Ganz besonders bevorzugt sind die Verbindungen der Formel (I), worin
- R: Methyl, Ethyl, Propyl, i-Propyl, t-Butyl, Phenyl, 2-Methoxy-ethyl oder Benzyl ist.

Alkyl kann verzweigt oder unverzweigt sein. Halogen steht für Cl, Br, I, bevorzugt für Cl. Unter Heteroaryl werden 5- bis 10 gliederige aromatische Ringe verstanden, die ein bis vier gleiche oder verschiedene Heteroatome aus der Reihe N, O, S enthalten, wie z.B. Pyrrol, Imidazol, Pyrazol, Pyridin, Pyrazin, Pyrimidin, Pyridazin, Furan, Thiophen, Oxazol, Isoxazol, Thiazol, Isothiazol, Triazol, Tetrazol, Triazin, Tetrazin, bevorzugt sind: Pyrrol, Imidazol, Oxazol, Thiazol und Pyridin.

Gegenstand der Erfindung ist ferner ein Verfahren zur Herstellung der Verbindungen der Formel (I) 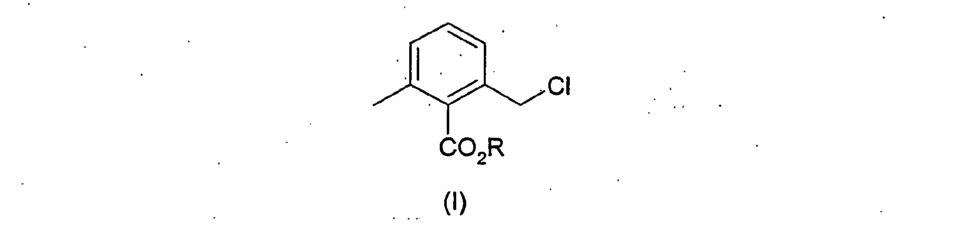 worin
- R: H, C₁-C₁₂-Alkyl, C₃-C₈-Cycloalkyl, C₆-C₁₂-Aryl, C₁-C₄-Alkyl-C₆-C₁₂-Aryl oder C₅-C₁₀-Heteroaryl bedeutet und wobei in Alkyl und Cycloalkyl ein oder mehrere CH₂-Gruppen durch -O- ersetzt und Alkyl, Cycloalkyl und Aryl durch Halogen substituiert sein können,
dadurch gekennzeichnet, dass man

Dimethylbenzoesäureester der Formel (II), 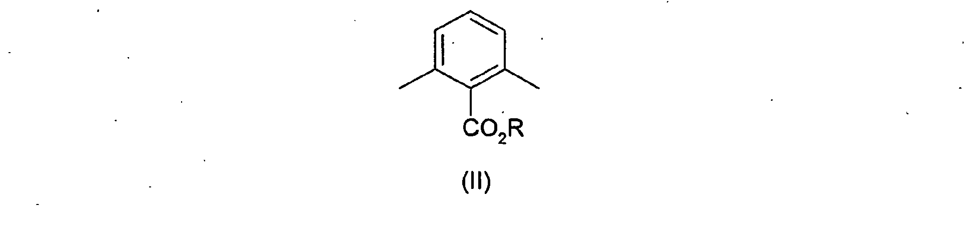 worin R wie oben definiert ist,
mit einem Chlorierungsreagenz, wie beispielsweise Sulfurylchlorid, N-Chlorsuccinimid (NCS), 1,3-Dichloro-5,5-dimethylhydantoin (NDDH) oder Trichlorisocyanursäure [Org Process Research&Development 2002,6,384-393], in einem inerten Lösungsmittel, wie beispielsweise CCl₄, Chlorbenzol oder ohne Lösungsmittel oberhalb 40°C umsetzt und anschließend gegebenenfalls reinigt.

Bevorzugt führt man die Reaktion bei 60 - 90 °C durch. Bei niedrigeren Temperaturen als 40 °C beobachtet man Chlorierung am Aromaten. Die Aufreinigung erfolgt bevorzugt destillativ oder über eine Kieselgelfiltration.

Die Chlorverbindungen der Formel (I) sind durch ringöffnende Chlorierung der Lactone (B) nicht oder nur sehr schlecht zugänglich, da das Lactongerüst sehr stabil ist. Ferner sind die 2-Chlormethylbenzoesäurederivate überraschenderweise destillierbar, können dabei in ausgezeichneter chemischer Reinheit isoliert werden und reagieren bei Lagerung nicht spontan zu den Lactonen.

Eine Umwandlung der Chlorverbindungen der Formel (I) in reaktivere Brom- oder Jodverbindungen kann ebenfalls von Vorteil sein, um die Reaktivität dieses Molekülbausteines bei der weiteren Synthese (beispielsweise zu den PPAR-Agonisten (C)) zu erhöhen. Diese Verbindungen weisen dann jedoch die in der Einleitung genannten "technischen" Nachteile auf. Die Umwandlung der Chlor in die Brom- bzw. Jodverbindung erfolgt mit Alkalihalogeniden in inerten Lösungsmitteln, bevorzugt mit Natriumbromid oder -iodid in Aceton am Rückfluss. Alternativ kann die Umhalogenierung und die weitere Synthese auch als Eintopfverfahren mit katalytischen oder stöchiometrischen Mengen Alkylhalogenid, bezogen auf die eingesetzte Chlorverbindung, durchgeführt werden.

Gegenstand der vorliegenden Erfindung ist weiterhin ein Verfahren zur Herstellung der Verbindungen der Formel (C), 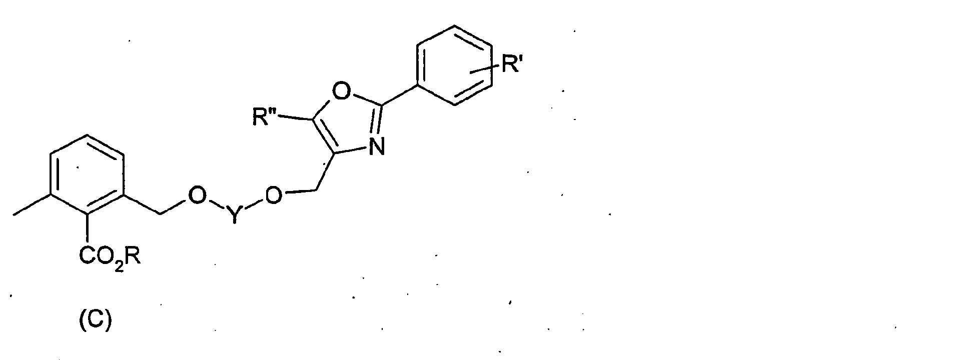 worin bedeuten
R H, C₁-C₁₂-Alkyl, C₃-C₈-Cycloalkyl, C₆-C₁₂-Aryl, C₁-C₄-Alkyl-C₆-C₁₂-Aryl oder C₅-C₁₀-Heteroaryl bedeutet und wobei in Alkyl und Cycloalkyl ein oder mehrere CH₂-Gruppen durch -O- ersetzt und Alkyl, Cycloalkyl und Aryl durch Halogen, substituiert sein können,
Y -(CH₂)₃-, 1,3-Phenylen, 1,3-Cyclohexandiyl;
R' H, F, Br, CF₃, (C₁-C₆)-Alkyl, O-(C₁-C₆)-Alkyl, Phenyl;
R" H, (C₁-C₆)-Alkyl, (C₁-C₃)-Alkyl-Phenyl, (C₅-C₆)-Cycloalkyl, Phenyl, CF₃;
dadurch gekennzeichnet, dass man Verbindungen der Formel (C1) 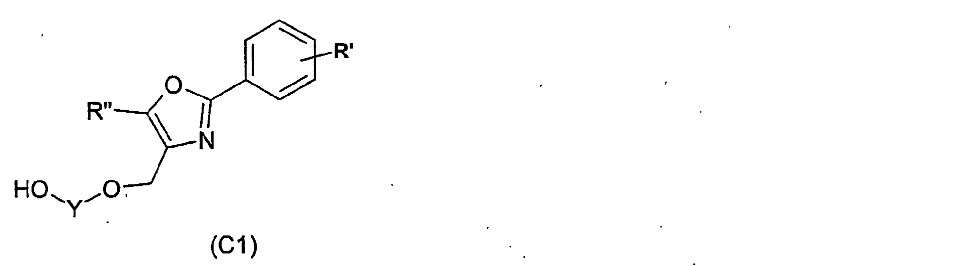 worin Y, R' und R" wie oben definiert sind,
mit Verbindungen der Formel (I) 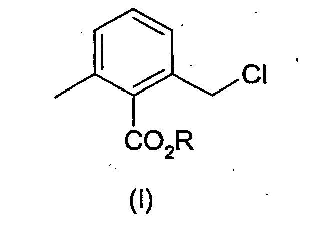 worin R wie oben definiert ist,
in Toluol, NMP oder anderen aprotischen Lösungsmitteln in Gegenwart einer geeigneten Base, bevorzugt mit Kalium-tert-butylat, bei -78 bis +50 °C, bevorzugt bei -30 bis +20 °C, umsetzt und anschließend extraktiv aufarbeitet und gegebenenfalls das Endprodukt kristallisiert.

Die Verbindungen der Formel (I) zeichnen sich durch eine hohe Stabilität gegenüber den entsprechenden Brom-Verbindungen aus. Vergleicht man die Stabilität von 2-Brommethyl-6-methylbenzoesäuremethylester mit der der analogen Chlor-Verbindung zeigt sich folgendes Ergebnis: 2-Chlormethyl-6-methylbenzoesäuremethylester lässt sich bei 66 - 77 °C / 0,1 bar unzersetzt destillieren, erst eine Sumpftemperatur von über 120 °C führt zu einer signifikanten Lactonbildung. Bei Raumtemperatur lässt sie sich über mehrere Monate stabil lagern. Die Lagerstabilität von 2-Brommethyl-6-methylbenzoesäuremethylester differiert hiervon deutlich. Bei Raumtemperatur nimmt der Gehalt der Bromverbindung innerhalb von wenigen Tagen rasch ab, innerhalb einer Woche von 92,6 auf 81,0%, innerhalb von 2 Wochen auf 67,8% und innerhalb von 2 Monaten auf 7,8%. Gleichzeitig steigt der Lactongehalt von 1,9% über 13,9% nach 1 Woche und 89,5% in 2 Monaten an.

Die folgenden Beispiele seien genannt.

### Beispiel 1:

### Synthese von 2-Chlormethyl-6-methylbenzoesäuremethylester

11.9 g 2,6-Dimethylbenzoesäuremethylester werden in 50 ml Chlorbenzol vorgelegt bei Raumtemperatur mit 8.2 g Sulfurylchlorid und 40 mg AIBN versetzt. Es wird 2 h bei 60-90°C gerührt. Danach wird mit 80 ml gesättigter NaHCO₃-Lösung versetzt. Nach der Phasentrennung wird die organische Phase mit 100 ml 10%iger Na2SO3-Lösung gewaschen, die organische Phase mit Magnesiumsulfat getrocknet und das Chlorbenzol i. Vak. abdestilliert. Es werden 15.5 g farblose Flüssigkeit erhalten. Das Produkt wird im Hochvakuum (0.1 mbar, 66-77°C) destilliert. Ausbeute: 10,2g (71 % d. Th.; 95,2 FI.-%).

### Beispiel 2:

### Synthese von 2-Chlormethyl-6-methylbenzoesäureisopropylester

19,2 g 2,6-Dimethylbenzoesäureisopropylester werden in 100 ml Tetrachlorkohlenstoff vorgelegt bei Raumtemperatur mit 13,3 g N-Chlorsuccinimid und 200 mg AIBN versetzt. Es wird 3 h am Rückfluss erhitzt. Nach dem Abkühlen wird abgesaugt und das Succinimid mit 20 ml Tetrachlorkohlenstoff gewaschen. Die Filtrate werden vereinigt und Tetrachlorkohlenstoff wird i. Vak. abdestilliert. Es werden 21,8 g farblose Flüssigkeit erhalten. Das Produkt wird im Hochvakuum (0.05 mbar, 94-97 °C) destilliert. Ausbeute: 13,9 g (61 % d. Th.; 93,6 FI.-%).

### Beispiel 3:

### Synthese von 2-Chlormethyl-6-methylbenzoesäure-2-methoxy-ethylester

10,4 g 2,6-Dimethylbenzoesäure-2-methoxy-ethylester werden bei Raumtemperatur mit 5,4 g Sulfurylchlorid und 40 mg AIBN versetzt. Es wird 1-2 h bei 60-90 °C gerührt. Danach wird mit 20 ml Wasser versetzt, die Phasen getrennt und die organische Phase mit Magnesiumsulfat getrocknet. Das Produkt wird im Hochvakuum (0,02 mbar, 95-103 °C) destilliert. Ausbeute: 6,4 g (66 % d. Th.; 91,8 FI.-%).

### Beispiel 4:

### Synthese von 2-Chlormethyl-6-methylbenzoesäurebenzylester

12,0 g 2,6-Dimethylbenzoesäurebenzylester werden in 50 ml Tetrachlorkohlenstoff vorgelegt bei Raumtemperatur mit 5,4 g Sulfurylchlorid und 40 mg AIBN versetzt. Es wird 4-5 h am Rückfluss gerührt. Danach wird mit 40 ml gesättigter NaHCO₃-Lösung versetzt. Nach der Phasentrennung wird die organische Phase mit 50 ml 10%iger Natriumsulfit-Lösung gewaschen und die organische Phase mit Magnesiumsulfat getrocknet. Die produkthaltige Lösung wird über Kieselgel filtriert und mit 20 ml Tetrachlorkohlenstoff nachgewaschen. Nach Abdestillieren des Lösungsmittels i. Vak. erhält man das Produkt als hellgelbes Öl. Ausbeute: 8,0g (73 % d. Th.; 88,4 FI.-%).

### Beispiel 5:

### 2-Methyl-6-[3-(2-phenyl-oxazol-4-ylmethoxy)-propoxymethyl]-benzoesäure

4.8 g 2-Chlormethyl-6-methylbenzoesäuremethylester werden in 250 ml Aceton bei Raumtemperatur gelöst und mit 35 g Natriumiodid versetzt. Die Mischung wird 6 h zum Rückfluss erhitzt. Anschließend wird das Solvent bei 0°C i. Vak. entfernt. Der Rückstand wird mittels LC-MS analysiert (87.7 FI.-% 2-lodmethyl-6-methylbenzoesäuremethylester) und in 20 ml Toluol gelöst. Die Lösung wird innerhalb von 10 min bei - 20°C zu einer Mischung von 5.0 g 3-(2-Phenyl-oxazol-4-ylmethoxy)-propan-1-ol, 4.8 g Kalium-tert.-butylat und 30 ml Toluol getropft. Danach wird 6 h bei - 20°C gerührt, mit 100 ml Wasser verdünnt und die wässrige Phase abgetrennt. Die organische Phase wird mit 40 ml NMP und 10 ml 32%iger Natronlauge versetzt und 8 h am Wasserabscheider zum Rückfluss erhitzt. Anschließend wird mit 100 ml Wasser versetzt und zweimal mit je 25 ml MTB-Ether extrahiert. Die wässrige Phase wird mit 5 ml Essigsäure angesäuert und zweimal mit je 50 ml Ethylacetat extrahiert. Nach dem Trennen der Phasen wird die organische Phase mit Magnesiumsulfat getrocknet und das Solvent i. Vak. entfernt. Nach der Kristallisation aus Diisopropylether erhält man 4.2 g 2-Methyl-6-[3-(2-phenyl-oxazol-4-ylmethoxy)-propoxymethyl]-benzoesäure (50 % d. Th., 99,2 HPLC-FI.-%)

## Patentansprüche

1. Verbindungen der Formel (I) 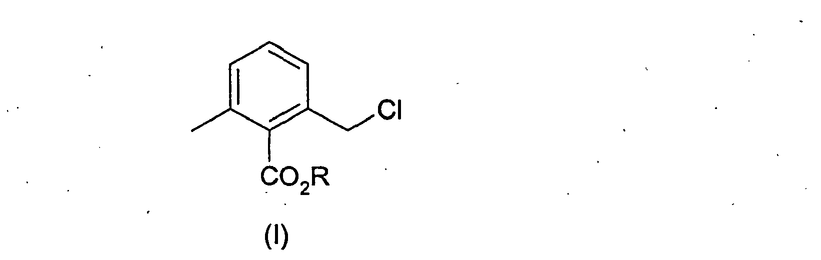 worin
R H, C₁-C₁₂-Alkyl, C₃-C₈-Cycloalkyl, C₆-C₁₂-Aryl, C₁-C₄-Alkyl-C₆-C₁₂-Aryl oder C₅-C₁₀-Heteroaryl bedeutet und wobei in Alkyl und Cycloalkyl ein oder mehrere CH₂-Gruppen durch -O- ersetzt und Alkyl, Cycloalkyl und Aryl durch Halogen, substituiert sein können.

2. Verbindungen der Formel (I) gemäß Anspruch 1, worin
R C₁-C₈ Alkyl, C₃-C₆-Cycloalkyl oder C₁-C₄-Alkyl-C₆-C₁₂-Aryl bedeutet, die gegebenenfalls durch Halogen substituiert sind und wobei ein oder zwei CH₂-Gruppen durch -O- ersetzt sein können.

3. Verbindungen der Formel (I) gemäß Anspruch 1 oder 2, worin
R C₁-C₆ Alkyl oder C₁-C₄-Alkyl-C₆-C₁₂-Aryl ist, die gegebenenfalls durch Halogen substituiert sind und wobei eine CH₂-Gruppe durch -O- ersetzt sein kann.

4. Verbindungen der Formel (I) gemäß den Ansprüchen 1 bis 3, worin
R Methyl, Ethyl, Propyl, i-Propyl, t-Butyl, Phenyl, 2-Methoxy-ethyl oder Benzyl ist.

5. Verfahren zur Herstellung der Verbindungen der Formel (I) gemäß den Ansprüchen 1 bis 4, **dadurch gekennzeichnet, dass** man Dimethylbenzoesäureester der Formel (II), 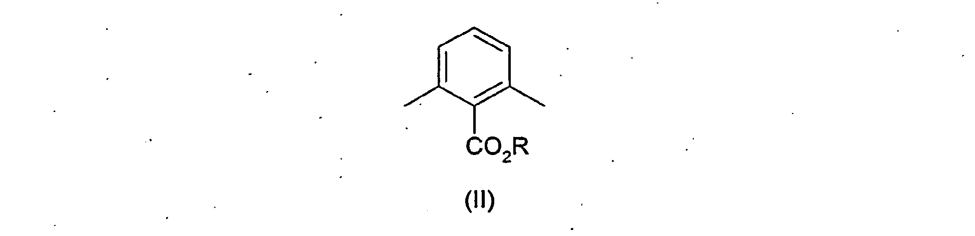 worin R wie oben definiert ist,
mit einem Chlorierungsreagenz in einem inerten Lösungsmittel oder ohne Lösungsmittel oberhalb 40°C umsetzt und anschließend gegebenenfalls reinigt.

6. Verfahren zur Herstellung der Verbindungen der Formel (C), 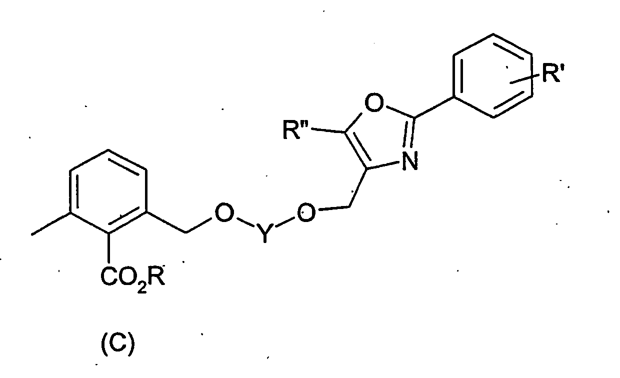 worin bedeuten
R H, C₁-C₁₂-Alkyl, C₃-C₈-Cycloalkyl, C₆-C₁₂-Aryl, C₁-C₄-Alkyl-C₆-C₁₂-Aryl oder C₅-C₁₀-Heteroaryl bedeutet und wobei in Alkyl und Cycloalkyl ein oder mehrere CH₂-Gruppen durch -O- ersetzt und Alkyl, Cycloalkyl und Aryl durch Halogen, substituiert sein können,
Y -(CH₂)₃-, 1,3-Phenylen, 1,3-Cyclohexandiyl;
R' H, F, Br, CF₃, (C₁-C₆)Alkyl, O-(C₁-C₆)-Alkyl, Phenyl;
R" H, (C₁-C₆)-Alkyl, (C₁-C₃)-Alkyl-Phenyl, (C₅-C₆)-Cycloalkyl. Phenyl, CF₃;
**dadurch gekennzeichnet, dass** man Verbindungen der Formel (C1) 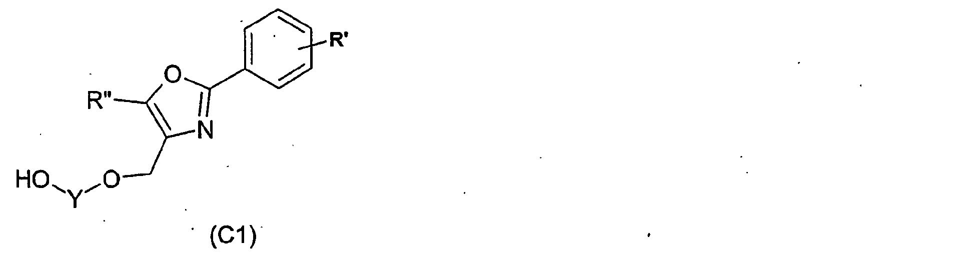 worin Y, R' und R" wie oben definiert sind,
mit Verbindungen der Formel (I) 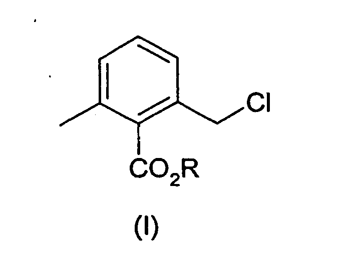 worin R wie oben definiert ist,
in Toluol, NMP oder anderen aprotischen Lösungsmitteln in Gegenwart einer geeigneten Base, bei -78 bis +50 °C umsetzt und anschließend extraktiv aufarbeitet und gegebenenfalls das Endprodukt kristallisiert.

7. Verfahren zur Herstellung der Verbindungen der Formel (C) gemäß Anspruch 6, worin der Phenylring durch R' in m- oder p-Stellung substituiert ist.

8. Verwendung der Verbindungen der Formel (I) gemäß den Ansprüchen 1 bis 4 zur Herstellung von PPAR-Agonisten der allgemeinen Formel (C).

## Claims

1. A compound of the formula (I) 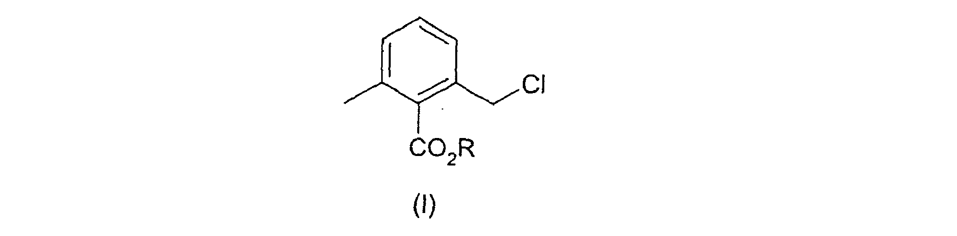 where
R is H, C₁-C₁₂-alkyl, C₃-C₈-cycloalkyl, C₆-C₁₂-aryl, C₁-C₄-alkyl-C₆-C₁₂-aryl or C₅-C₁₀-heteroaryl, and, in alkyl and cycloalkyl, one or more CH₂ groups may be replaced by -O-, and alkyl, cycloalkyl and aryl may be substituted by halogen.

2. A compound of the formula (I) as claimed in claim 1 in which
R is C₁-C₈ alkyl, C₃-C₆-cycloalkyl or C₁-C₄-alkyl-C₆-C₁₂-aryl, each of which may optionally be substituted by halogen and in which one or two CH₂ groups may be replaced by -O-.

3. A compound of the formula (I) as claimed in claim 1 or 2 in which
R is C₁-C₆ alkyl or C₁-C₄-alkyl-C₆-C₁₂-aryl, each of which may optionally be substituted by halogen and in which one CH₂ group may be replaced by -O-.

4. A compound of the formula (I) as claimed in claims 1 to 3 in which
R is methyl, ethyl, propyl, i-propyl, t-butyl, phenyl, 2-methoxyethyl or benzyl.

5. A process for preparing the compounds of the formula (I) as claimed in claims 1 to 4, which comprises
reacting dimethylbenzoic esters of the formula (II) 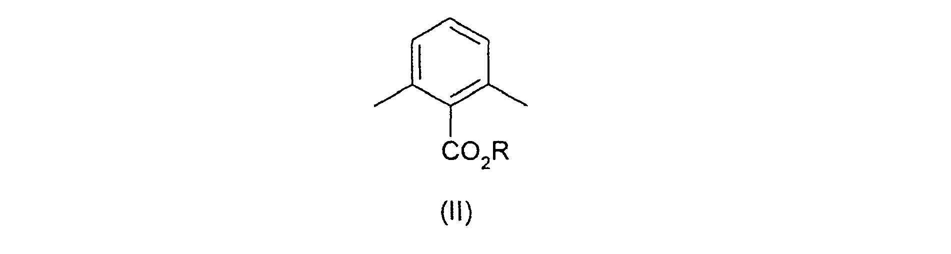 where R is as defined above
with a chlorinating reagent in an inert solvent or without solvents above 40°C and subsequently optionally purifying.

6. A process for preparing the compounds of the formula (C) 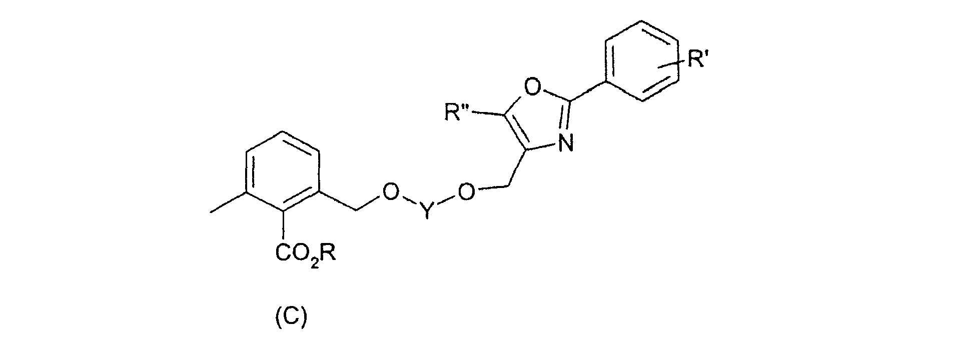 in which
R is H, C₁-C₁₂-alkyl, C₃-C₈-cycloalkyl, C₆-C₁₂-aryl, C₁-C₄-alkyl-C₆-C₁₂-aryl or C₅-C₁₀-heteroaryl and, in alkyl and cycloalkyl, one or more CH₂ groups may be replaced by -O- and alkyl, cycloakyl and aryl may be substituted by halogen,
Y is -(CH₂)₃-, 1,3-phenylene, 1,3-cyclohexanediyl,
R' is H, F, Br, CF₃, (C₁-C₆)-alkyl, O-(C₁-C₆)-alkyl, phenyl;
R" is H, (C₁-C₆)-alkyl, (C₁-C₃)-alkylphenyl, (C₅-C₆)-cycloalkyl, phenyl, CF₃;
which comprises reacting compounds of the formula (C1) 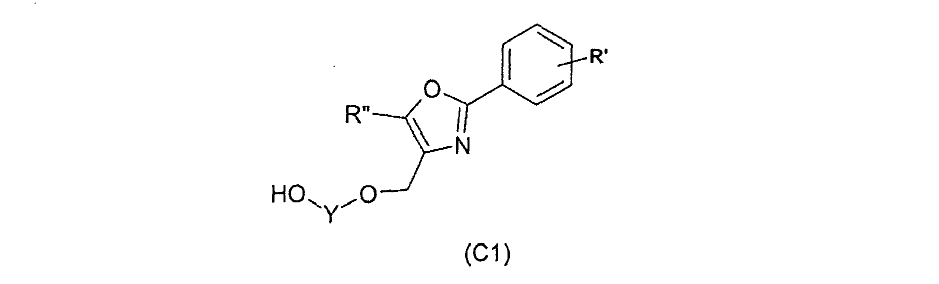 where Y, R' and R" are each as defined above
with compounds of the formula (I) 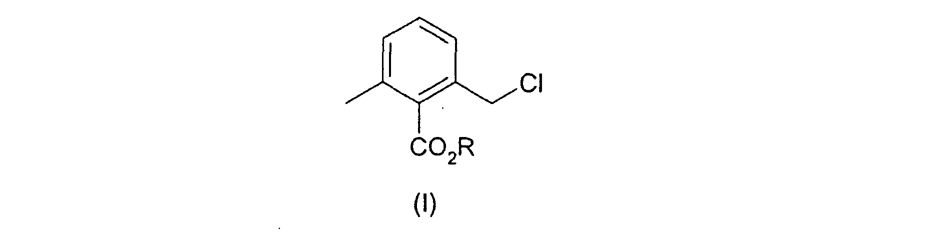 where R is as defined above
in toluene, NMP or other aprotic solvents, in the presence of a suitable base, at -78 to +50°C, and subsequently working up extractively and optionally crystallizing the end product.

7. The process for preparing the compounds of the formula (C) as claimed in claim 6, wherein the phenyl ring is substituted by R' in the m- or p-position.

8. The use of the compounds of the formula (I) as claimed in claims 1 to 4 for preparing PPAR agonists of the general formula (C).

## Revendications

1. Composés de formule (I) 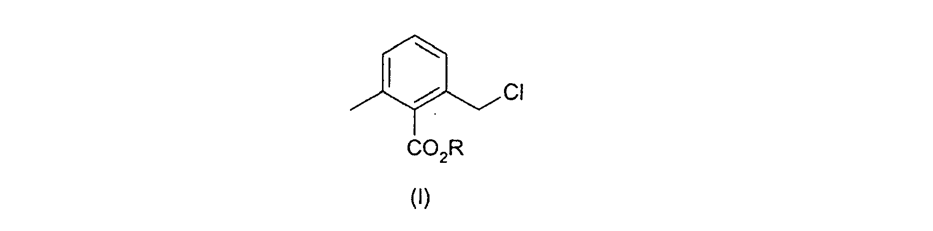 dans laquelle :
R représente un atome d'hydrogène, un groupe alkyle en C₁-C₁₂, un groupe cycloalkyle en C₃-C₈, un groupe aryle C₆-C₁₂, un groupe C₁-C₄-alkyl-C₆-C₁₂-aryle ou un groupe hétéroaryle en C₅-C₁₀, et où, dans un groupe alkyle et un groupe cycloalkyle, un ou plusieurs groupes CH₂ peuvent être remplacés par un groupe -O-, et un groupe alkyle, un groupe cycloalkyle et un groupe aryle peuvent être substitués par un atome d'halogène.

2. Composés de formule (I) selon la revendication 1, dans laquelle :
R représente un groupe alkyle en C₁-C₈, un groupe cycloalkyle en C₃-C₆ ou un groupe C₁-C₄-alkyl-C₆-C₁₂-aryle, qui sont éventuellement substitués par un atome d'halogène et où un ou deux groupes CH₂ peuvent être remplacés par un groupe -O-.

3. Composés de formule (I) selon la revendication 1 ou 2, dans laquelle :
R représente un groupe alkyle en C₁-C₆ ou un groupe C₁-C₄-alkyl-C₆-C₁₂-aryle, qui sont éventuellement substitués par un atome d'halogène et où un groupe CH₂ peut être remplacés par un groupe -O-.

4. Composés de formule (I) selon les revendications 1 à 3, dans laquelle :
R représente un groupe méthyle, un groupe éthyle, un groupe propyle, un groupe isopropyle, un groupe t-butyle, un groupe phényle, un groupe 2-méthoxyéthyle ou un groupe benzyle.

5. Procédé de préparation des composés de formule (I) selon les revendications 1 à 4, **caractérisé en ce que** :
des esters de l'acide diméthylbenzoïque de formule (II), 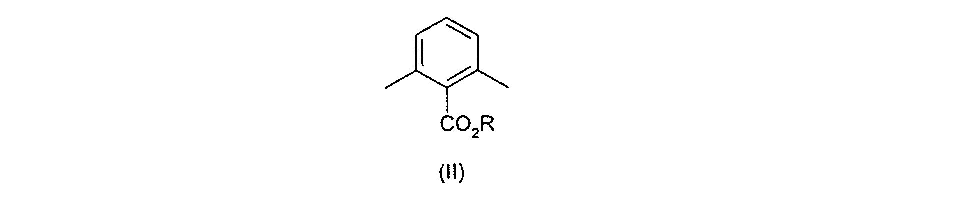
dans laquelle R est tel que défini ci-dessus, sont mis à réagir avec un réactif de chloration, dans un solvant inerte ou sans solvant, au-dessus de 40°C et sont ensuite éventuellement purifiés.

6. Procédé de préparation des composés de formule (C), 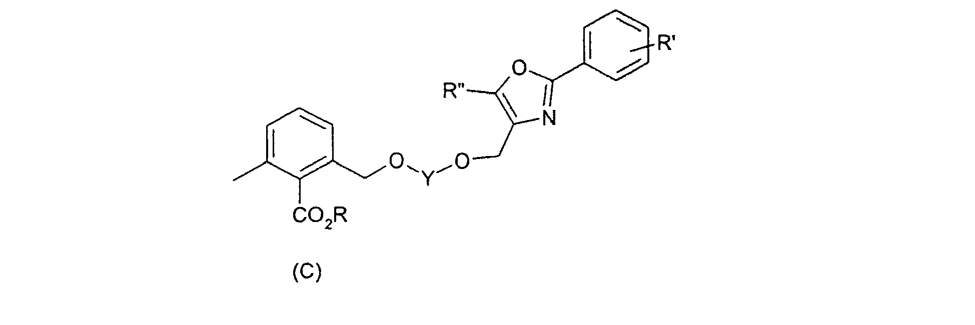 dans laquelle :
R représente un atome d'hydrogène, un groupe alkyle en C₁-C₁₂, un groupe cycloalkyle en C₃-C₈, un groupe aryle C₆-C₁₂, un groupe C₁-C₄-alkyl-C₆-C₁₂-aryle ou un groupe hétéroaryle en C₅-C₁₀, et où, dans un groupe alkyle et un groupe cycloalkyle, un ou plusieurs groupes CH₂ peuvent être remplacés par un groupe -O-, et un groupe alkyle, un groupe cycloalkyle et un groupe aryle peuvent être substitués par un atome d'halogène ;
Y représente un groupe -(CH₂)₃-, un groupe 1,3-phénylène, un groupe 1,3-cyclohexane-diyle ;
R' représente un atome d'hydrogène, un atome de fluor, un atome de brome, un groupe CF₃, un groupe alkyle en C₁-C₆, un groupe O-C₁-C₆-alkyle, un groupe phényle ;
R'' représente un atome d'hydrogène, un groupe alkyle en C₁-C₆, un groupe C₁-C₃-alkylphényle, un groupe cycloalkyle en C₅-C₆, un groupe phényle, un groupe CF₃ ;
**caractérisé en ce que** des composés de formule (C1), 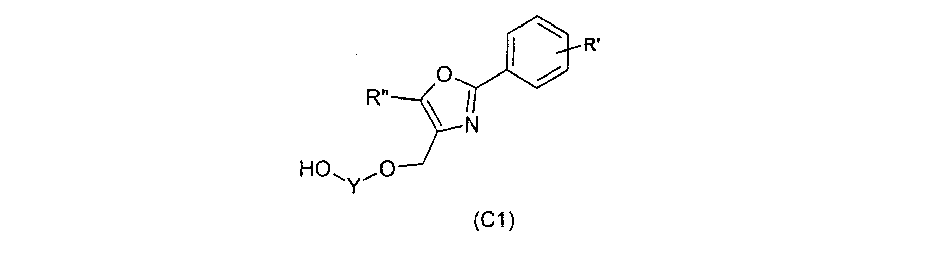 dans laquelle Y, R' et R" sont tels que définis ci-dessus,
sont mis à réagir avec des composés de formule (I), 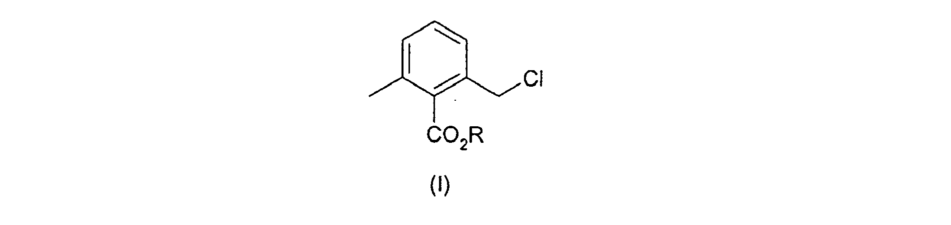 dans laquelle R est tel que défini ci-dessus,
dans du toluène, de la NMP ou d'autres solvants aprotiques, en présence d'une base appropriée, à entre - 78 et + 50°C, et on procède ensuite au traitement par extraction et on cristallise éventuellement le produit final.

7. Procédé de préparation des composés de formule (C) selon la revendication 6, dans laquelle le noyau phényle est substitué par R' en position m ou p.

8. Utilisation des composés de formule (I) selon les revendications 1 à 4 pour la préparation d'agonistes de PPAR de formule générale (C).
